# EUROPEAN PATENT APPLICATION

(11) **EP 4 744 572 A1**
(43) Date of publication of application: **20.05.2026**
(21) Application number: 24212640.7
(22) Date of filing: 13.11.2024
(51) Int. Cl.: A61B 1/00, A61B 1/05, A61B 1/267

(54) **VIDEO LARYNGOSCOPE**

(71) Applicant: Kumar, Sujit, Redmond, WA 98052 (US)
(72) Inventor: Kumar, Sujit, Redmond, WA 98052 (US)
(74) Representative: Dr. Gassner & Partner mbB

(57) **Abstract**

The present invention provides a video laryngoscope (100) to perform the intubation process. The video laryngoscope (100) includes a handle (10), a blade (20), a display unit (30), and an attachment assembly (25). The handle (10) includes a camera housing (16) with an image sensor (161) and a light source (162) arranged therein. The image sensor (161) is attached to the handle (10) through a camera channel (15) to visualise the path of the endotracheal tube. The camera housing (16) has a deflector (18) to avoid the blurry and distorted visuals generated due to the scattering of the light from the light source (162). The blade (20) is arranged on the handle (10) to cover the handle (10) and the camera channel (15). Further, the display unit (30) is provided for better visibility of the display screen (34) to visualise the path of the endotracheal tube.

## Description

### TECHNICAL FIELD

The present invention relates to a laryngoscopy device. More particularly, the present invention relates to a video laryngoscope.

### BACKGROUND

A laryngoscope is a device, typically comprising a handle and a blade, which is used by clinicians during endotracheal intubation. The laryngoscope assists with intubation by allowing the clinician to visualise the path of the endotracheal tube as the laryngoscope passes through the glottis towards the trachea.

Presently, traditional video laryngoscopes come in two form factors. In the first form factor, the display is detached from the handle and is attached to a cart. The display attached to the cart has a limited manoeuvrability. Also, the size of the display is 7-11 inches and the carts are bulky and hard to move around. While performing the intubation process, the clinician needs to look away from the patient and towards the display which is attached to the cart and not looking at the patient increases the risk of a failed process. In addition, cart-based systems tend to be more expensive and hospitals purchase a few units to be shared between multiple operating rooms. This results in the video laryngoscope not being available for every intubation procedure. In the second form factor, the display is attached to the handle but the displays are small, have poor image quality and are not manoeuvrable. Both form factors result in increased risk to the patient and repeated intubations.

Further, traditional video laryngoscope includes reusable blades that are used again and again for the intubation process. Repeated use of the blades leads to a higher risk of infection, increased operational burden on hospitals and high ownership costs.

Traditional video laryngoscopes often employ complex or cumbersome attachment systems between the display unit, handle, and blade. These mechanisms, which may include threaded connections, magnetic fasteners, or bayonet mounts, can be difficult to operate, especially in high-pressure medical scenarios such as emergency intubations. One major issue with these attachment systems is their potential for misalignment or wear over time. Repeated assembly and disassembly of the components can degrade the precision of the connection, leading to unreliable performance during critical procedures. For example, improper alignment between the display and the handle may result in loose or unstable fittings, which can disrupt the clinician's workflow, causing delays and increasing the risk of patient complications.

Moreover, many current video laryngoscope designs fail to provide flexibility in the positioning and orientation of the display unit. These units are often fixed in place, forcing clinicians to adjust their body position or viewing angle to align with the screen. In scenarios where rapid visualization of the airway is critical, such limitations can hinder the efficiency of the procedure. Furthermore, the inability to adjust the orientation of the video feed can make it difficult to obtain a clear view of the airway, particularly in challenging intubations or in patients with anatomical variations.

Existing designs also tend to lack user-friendly control interfaces, often requiring clinicians to interact with multiple buttons to operate the display, adjust settings, or capture images. This complexity can lead to interruptions during the procedure, as clinicians must frequently pause to manipulate the device, taking their focus away from the patient.

Therefore, there is a need for a video laryngoscope, which overcomes few or all drawbacks of the prior art.

### STATEMENT OF THE INVENTION

According to the present aspect of the invention, there is provided a video laryngoscope. The video laryngoscope includes a handle designed to allow a clinician to hold the laryngoscope, with an upper end and a lower end. The handle may feature a display unit attachable to the upper end through an attachment assembly, while a blade may be removably attached around the handle and a camera channel extending from the lower end. The camera channel may have a camera housing to capture visuals of the path of the endotracheal tube, larynx, trachea, or airway.

In some embodiments, the attachment assembly may connect the display unit to the handle through a hinge mechanism, enabling 360-degree rotation of the display unit to provide optimal viewing angles during intubation. The blade may be attached to the handle via a dual ball mechanism, where two ball projections extend radially outward and engage with openings in the blade, holding the blade in place until the ball projections are pressed inward for detachment.

The display unit may include a touch user interface, enabling clinicians to interact with stored images and videos, configure settings such as brightness, sound, and output port controls, and review captured visual data. The display unit may also establish a wireless connection with the handle, allowing it to display visuals captured by the image sensor even when detached.

In another embodiment, the handle may be adapted to receive both adult and pediatric blades without requiring additional adjustments, making it suitable for various patient types. The display unit may include a display screen for real-time visuals, and a power button to turn the unit ON or OFF. The video laryngoscope may include a memory and processor to store and process captured images and videos, as well as an output port for connecting to external displays.

The display unit may also include a feature for flipping or rotating the displayed visuals, controlled through a touchscreen or an arrow icon. For enhanced data security, a passcode may be required to access stored visuals or to connect the display unit to external devices, such as PCs, Macs, or tablets.

In a further embodiment, the system may include an electrical connection established between the camera housing and the display unit when the attachment assembly is mounted on the handle, enabling live transmission of visuals from the camera channel to the display screen.

Another aspect of the invention may provide an attachment assembly with dual projections arranged on the attachment assembly housing. These projections may engage with corresponding openings in the receiving port of the handle, securing the assembly until the projections are pressed inward for detachment. The dual projections may include ball ends and protruded sleeves that engage with the handle to securely attach the display unit.

Additionally, the video laryngoscope may feature a display unit that automatically starts displaying visuals from the image sensor upon being powered on, eliminating the need for pressing a record button to initiate the display of live visuals. The display unit may also be equipped with a record button to capture images or videos during the procedure by pressing or holding the button, respectively.

In another embodiment, the system may incorporate passcode protection to access stored images or videos, ensuring secure data management. The system may also provide an automatic connection between the camera channel and display unit, allowing for seamless image transmission when the display unit is attached to the handle

An object of the present invention is to provide a video laryngoscope.

Another object of the present invention is to provide a video laryngoscope, which is adapted to control the infection rate in hospitals.

Yet another object of the present invention is to provide a video laryngoscope which is adapted to capture clear and non-distorted images of the path of the endotracheal tube, larynx, trachea or airway.

Still another object of the present invention is to provide a video laryngoscope which provides better visibility of the path of the endotracheal tube to the clinicians.

### BRIEF DESCRIPTION OF THE DRAWINGS

Other features and advantages of the invention will become apparent when reading the detailed description given below, purely by way of example and in a non-limitative manner, referring to the following figures:
FIG. 1 illustrates a side view of a video laryngoscope in accordance with the present invention;
FIG. 2a, and 2b illustrate a front view and a top view of a handle of the video laryngoscope in accordance with the present invention;
FIG. 3 illustrates a perspective view of a camera housing of the video laryngoscope in accordance with the present invention;
FIG. 4 illustrates a perspective view of a deflector of the video laryngoscope in accordance with the present invention;
FIG. 5 illustrates a front view of a display assembly of the video laryngoscope in accordance with the second embodiment the present invention;
FIG. 6 illustrates a side view of a video laryngoscope in accordance with the embodiment illustrated in FIG. 5;
FIG. 7 illustrates a side view of a video laryngoscope in accordance with a third embodiment of the invention;
FIG. 8 illustrates a front view of a display unit of a video laryngoscope in accordance with the embodiment shown in FIG. 7; and
FIG. 9 illustrates a bottom view of a display cover of the video laryngoscope in accordance with the present invention;

### DETAILED DESCRIPTION

An embodiment of this invention, illustrating its features, will now be described in detail. The words "comprising, "having, "containing," and "including," and other forms thereof, are intended to be equivalent in meaning and be open ended in that an item or items following any one of these words is not meant to be an exhaustive listing of such item or items, or meant to be limited to only the listed item or items.

The present invention provides a video laryngoscope. The video laryngoscope is adapted to seamlessly attach and detach a display unit and a blade from a handle. The video laryngoscope is adapted to avoid the blurry and distorted visuals of the path of the endotracheal tube. Further, the video laryngoscope is adapted to control the infection rate which is spread due to the regular laryngoscopes. Furthermore, the video laryngoscope allows the clinicians to perform the intubation process without sacrificing the visibility of the display unit.

The terms "first," "second," and the like, herein do not denote any order, quantity, or importance, but rather are used to distinguish one element from another, and the terms "an" and "a" herein do not denote a limitation of quantity, but rather denote the presence of at least one of the referenced item. The disclosed embodiments are merely exemplary of the invention, which may be embodied in various forms.

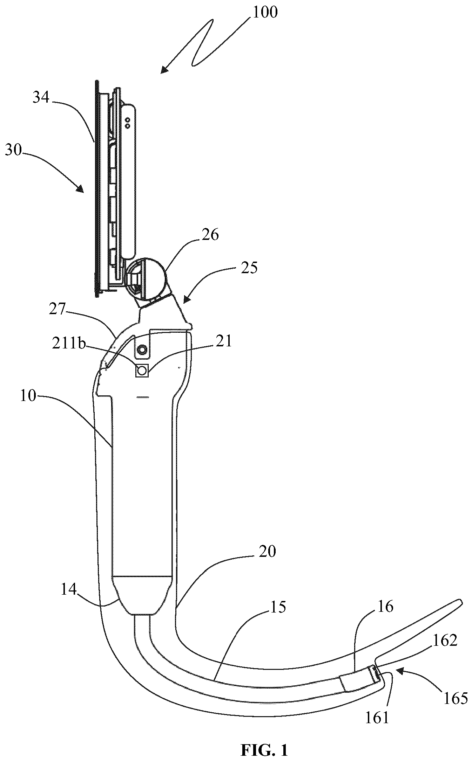

Referring now to FIG. 1, a video laryngoscope (100) in accordance with the present invention is illustrated. referring now to the video laryngoscope (100) as an "laryngoscope (100)". The laryngoscope (100) includes a display unit (30), an attachment assembly (25) and a handle (10).

The display unit (30) is configuring to for show visuals of the path of the endotracheal tube, larynx, trachea, or airway. The display unit (30) is made of rectangular design having a protective edge made of the flexible material to protect the display unit (30) from the damage. It is obvious for the person skilled in the art to configure display unit (30) of any shape or material as per the requirement. The display unit (30) includes a display screen (34) on the centre of the display unit (30). The display screen (34) is an LCD screen made of a liquid crystal material, the display screen (34) visualises an images and videos through the conductors and electrode pattern, specifically the display screen (34) displays the visuals of the path of the endotracheal tube, larynx, trachea, or airway. The person skilled in the art can configure screen made of a quantum dot display in an embodiment. In another embodiment an LED backlit LCD may be arranged within display screen (34). In another embodiment a WLCD, OLCD, Plasma, or OLED or its derivatives (Transparent OLED, PMOLED, AMOLED) or a micro-LED or a like. It is obvious for the person skilled in the art to configure any element or mechanism to show the visuals to the clinician.

Referring now to FIG. 1, 3, and 5, the display unit (30) includes a battery (not shown) to power up the display unit (30) while using. The battery can be a replaceable battery or a rechargeable battery allowing to reuse or replace the battery upon requirement. The display unit (30) further includes a power button (32) allowing the clinician to power ON and OFF the display unit (30). The power button (32) is arranged on the lower side of the protective edge of the display unit (30), the position of the power button (32) is within the finger range of the clinician allowing the clinician to operate the power button (32) with only single hand. It is obvious for the person skilled in the art to configure the power button (32) anywhere in the display unit (30) allowing to the clinician to operate the power button (32) with only one hand.

The display unit (30) further includes an output port (33). The output port (33) is provided on any of the sides of the display unit (30) to connect the display unit (30) to an external display (not shown) to show the visuals of the path of the endotracheal tube. The external display has a bigger screen size than the display screen (34) of the display unit (30). It is obvious to a person skilled in the art to provide a plurality of output ports on the display unit (30) to allow the clinician to connect the display unit (30) with plurality of the external display or use the output port as an USB or charging port to charge the battery of the display unit (32).

The display unit (30) further includes a processor (not shown), and a memory (not shown) to process and store the visuals of the path of the endotracheal tube, larynx, trachea or airway. The processor processes all the function of the laryngoscope (100) including all the commands given by the clinician. The memory stores the files for future reference of the clinician. The person skilled in the art can configure any processor as per the requirement and also arrange the memory with any capacity according to the requirement.

The display screen (34) further includes a touch user interface (TUI) (not shown) integrated within the display screen (34). The display screen (34) allows the clinician to interact with the laryngoscope (100). The touch user interface of the display screen (34) is adapted to review and manage previously recorded images or videos. The touch user interface includes a home page (not shown) to show the current time and date, a menu icon (not shown) to access the plurality of icons, and a battery icon (not shown) to indicate the level of the battery. The display screen (34) is powered on using the power button (32) to display the home page. The clinician interacts with the menu icon of the home page by touching the menu icon displayed on the display screen (34) to access the plurality of the icons. The menu icon is provided on the bottom left corner of the display screen (34) to allow the clinician to reach the menu icon with one hand.

In the present embodiment of the present invention, the display unit (30) includes a charging light (35) arranged on the top of protective edge of the display unit (30) to indicate the charging status of the battery. The charging light (35) turns ON when the battery is connected to a power source while recharge the battery, and turns OFF when the battery is not connected to the power source for recharging. It is obvious to a person skilled in the art to arrange the charging light (35) or any other element for indicating the charging status of the battery and can arranged that element to any of the corners of the display unit (30).

The display unit (30) further includes a security passcode (not shown), provide security to the previously stored file in the memory of the display unit (30). The security passcode required for opening the file icon of the touch user interface. The clinician needs to put a key passcode in the security passcode in order to access the stored files within the memory. Further the security passcode also required upon connecting the display unit (30) with external screen through the output port. The laryngoscope (100) initially comes with default passcode which is "000000", the clinician can set the any passcode within the 6 digits upon opening the setting icon of the security passcode.

The display screen (34) also includes an arrow (not shown) for adjusting the visuals orientation while using the laryngoscope (100), the adjustable arrow feature allows to flipping visuals orientation upside down as per the requirement of the situation. The clinician can interact with the display screen (34), and an arrow directing the adjustable direction arrange on a top and bottom of the display screen (34). Upon interaction with the arrow, the commands go to the processor and the processor processes the commands and the flip the display screen (34) visual vertically upside down. It is obvious for the person skilled in the art to configure any other icon or element to adjust the visuals of the display screen (34) with any other icon not limited to the arrow, at any angle without disturbing the orientation of the display unit (30).

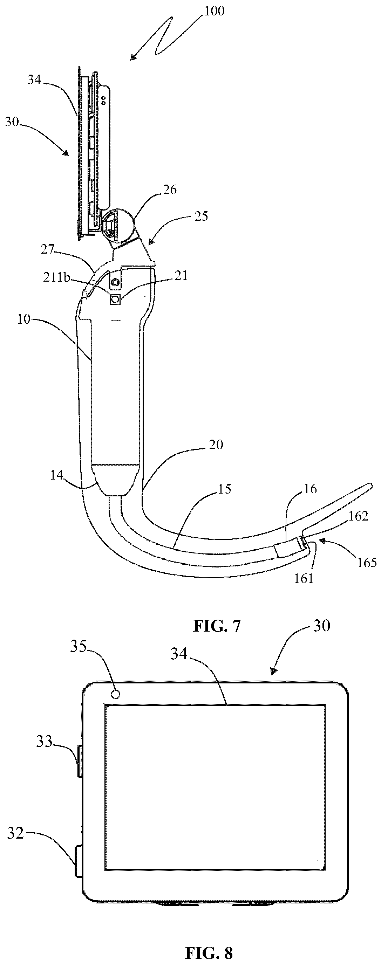

As shown in FIG. 1 and 7, a display cover (40) is provided to prevent the display unit (30) and the attachment assembly (25) from getting infected during the intubation process. The display cover (40) is made up of a transparent material to allow the clinician to view the display screen (34) without any hindrance. The display cover (40) includes a lower panel (42) and an upper panel (44) hinged at a hinge point. The lower panel (42) and the upper panel (44) pivot around the hinge point to cover the display unit (30). The upper panel (44) and the lower panel (42) of the display cover (40) are pressed to snap fit with each other after placing the display unit (30) within a cavity of the display cover (40). Further, the upper panel (44) has a flexible sheath (not shown) extending from the free end of the upper panel (44) to cover the attachment assembly (25) and a portion of the handle (10). The display cover (40) is disposable and can be disposed of once the intubation process is finished. In the present embodiment the display cover is solid transparent material to protect the display unit with unwanted scratch or damage. It is obvious for the person skilled in the art to configure the display cover (40) with any colour or with any material or solid or flexible as per the requirement.

In another embodiment of the present invention, and the display cover (40) may be made of flexible transparent material allowing the clinician to cover and uncover the display unit (30) by stretch-ably placing the flexible transparent cover on the display unit (30). This flexible display cover does not have any snap fit arrangement, it can be manually place on the display unit (30).

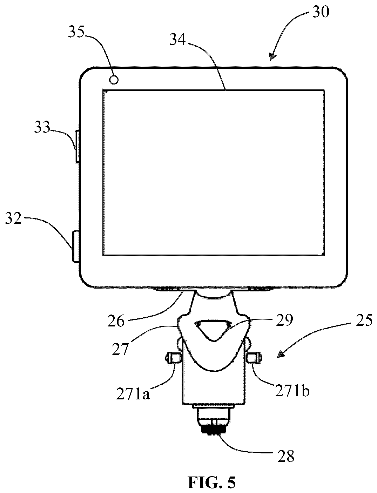

Referring now to FIG. 1, and 5, the display unit (30) which is attachable to the handle (10) through the attachment assembly (25). The attachment assembly (25) includes a housing (27), and a second terminal (28) which is electrically connected to the display unit (30). The housing (27) is connected to the display unit (30) using a hinge (26). The hinge (26) is provided to pivot the display unit (30) in a back-and-forth direction. The hinge (26) is connected to the display unit (30) and the housing (27).

The handle (10) is configured to allow a clinician to hold the laryngoscope (100), the handle (10) has an upper end and a lower end. The upper end of the handle (10) is provided with a receiving port (12) with two openings (121a, 121b) opposite to each other to receive the attachment assembly (25). The handle (10) is an elongated member that has an ergonomic design that allows clinicians to easily hold the handle (10) during an intubation process.

Further, the housing (27) includes dual projections (271a, 271b) extending from an outer surface of the housing (27). Each projection (271a, 271b) is arranged opposite to each other having a compressible spring element (not shown) between each positioned within the housing (27). The dual projections (271a, 271b) securely attach and detach the housing (27) to the handle (10). The dual projections (271a, 271b) are biased to radially extend away from the centre axis of the housing (27).

The housing (27) is attached to the receiving port (12) of the handle (10). Specifically, the receiving port (12) has two openings (121a, 121b) to receive the respective dual projections (271a, 271b) of the housing (27) to securely hold the housing (27) within the receiving port (12) of the handle (10). The dual projections (271a, 271b) ensure that the housing (27) is held in place until the dual projections (271a, 271b) are pressed to detach the housing (27) of the attachment assembly (25). The diameter of the handle (10) is bigger than the diameter of the housing (27), to securely receive the hosing (27) within an upper end of the handle (10).

Once the display unit (30) is attached to the handle (10) through the attachment assembly (25), the second terminal (28) of the attachment assembly (25) electrically connects with the handle (10) to establish the electric connection between the display unit (30) and the handle (10). Specifically, upon attaching the attachment assembly (25) to the handle (10), the second terminal (28) immediately establish an electric connection between the display unit (30) enabling the instantaneous display of visuals of the path of the endotracheal tube, larynx, trachea, or airway on the display screen (34).

Furthermore, the attachment assembly (25) includes a record button (29) (as shown in FIG. 5) to capture an image or record a video. In the present embodiment of the invention, the record button (29) is arranged on a portion of the housing (27). However, the person skilled in the art can arrange the record button (29) on any other portion in such a way that the clinician's thumb easily reaches to the record button (29) to capture an image or record a video.

The touch user interface is adapted to show an indication of the captured image on the display screen (34) when the record button (29) is pressed by the clinician to capture the image. Further, the touch user interface is adapted to show a timer of the recording on the display screen (34) when the record button (29) is pressed and held to record the video. Further, the timer disappears from the display screen (34) after pressing the record button (29) to stop the recording. In an embodiment, the record button (29) may be integrated with multiple function not limited to record the image or video. The person skilled in the art can configure the record button (29) at any position on the display unit (30) having multiple features as per the requirement.

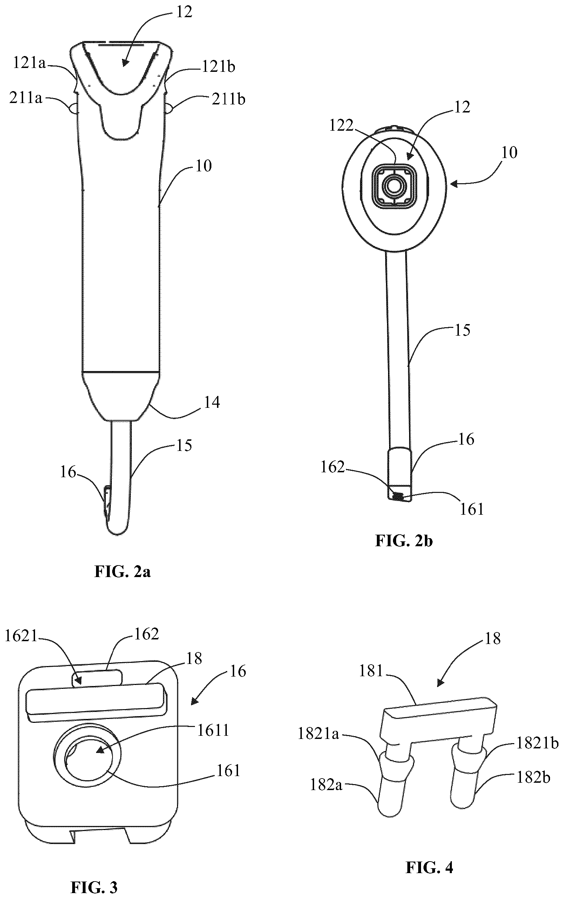

The lower end of the handle (10) is provided with a tapered portion (14). Specifically, the lower end of the handle (10) is provided with a camera channel (15) that extends from the tapered portion (14) of the handle (10). The camera channel (15) is a flexible link extending from the tapered portion (14) of the handle (10) and comprises an electric circuit to transfer the visuals like images and videos to the receiving port (12). Specifically, the camera channel (15) is electrically connected to a first terminal (122) of the receiving port (12) arranged between the lower end and the top end of the tapered portion (as shown in FIG. 2b). The camera channel (15) has a first end connected to the tapered portion (14) of the handle (10) and a second end comprising a camera housing (16). The camera housing (16) of the camera channel (15) is provided to capture visuals of the path of the endotracheal tube, larynx, trachea or airway.

Referring now to FIG. 1, and 3, the camera housing (16) is in the square shape includes an image sensor (161) and a light source (162). It is obvious for the person skilled in the art to configure the camera housing (16) with any shape and size as per the requirement. In the present embodiment, the camera housing (16) includes one image sensor (161) and one light source (162). The person skilled in the art can arranged any number of image sensor (161) and light source (162) as per the requirement. The image sensor (161) facilitates capturing of the visuals of the path of the endotracheal tube, larynx, trachea or airway. Specifically, an image sensor opening (1611) in the camera housing (16) allows the image sensor (161) to capture the visuals of the path of the endotracheal tube, larynx, trachea or airway. In the present embodiment of the invention, the image sensor (161) is a 2-megapixel (MP) camera, but the person skilled in the art can use any other image sensor (161) having different specifications to capture the visuals of the path of the endotracheal tube, larynx, trachea or airway.

In the present embodiment, the display unit (30) is configured to establish a wireless connection with the handle (10), allowing the display unit (30) to display visuals from the image sensor (161) even when detached from the handle (10). It is obvious for the person skilled in the art to connect the display unit (30) with the image sensor (161) by configuring any mechanism or connection as per the requirement.

Further, the camera housing (16) includes a light source opening (1621) to allow the light source (162) to illuminate the path of the endotracheal tube and the airway. Furthermore, the camera housing (16) has a deflector (18). Specifically, the deflector (18) is arranged on an outer surface of the camera housing (16) separating the image sensor opening (1611) and the light source opening (1621). The deflector (18) avoids the direct exposure of the light source (162) to the image sensor (161).

In the present embodiment of the invention, the deflector (18) is a rectangular strip. The rectangular strip is made up of a silicone material. A person skilled in the art can configure the deflector (18) with any other material, shape or size according to the size and position of the image sensor (161) and the light source (162). The deflector (18) is horizontally arranged between the light source opening (1621) and the image sensor opening (1611) to avoid the scattering of the light on the image sensor (161).

Referring now to FIG. 3, and 4, the deflector (18) includes a rectangular portion (181), and two legs (182a, 182b) extending from the ends of the rectangular portion (181). The two legs (182a, 182b) are provided with respective locking elements (1821a, 1821b) to attach the deflector (18) to the camera housing (16). Specifically, the camera housing (16) has two openings (not shown) to receive the locking elements (1821a, 1821b) of the two legs (182a, 182b) to hold the deflector (18) on the outer surface of the camera housing (16). The deflector (18) is fixedly attached to the camera housing (16) using the locking elements (1821a, 1821b). The two legs (182a, 182b) have cylindrical shape extending from the ends of the rectangular portion (181). The shape of the locking element (1821a, 1821b) in a way that the diameter of the locking element facing the rectangular portion (181) is bigger and the other end of the locking element (1821a, 1821b) has the same diameter as the two legs (182a, 182b). The two legs (182a, 182b) and the locking elements (1821a, 1821b) are made of the flexible material, upon pressing the deflector (18) within the opening of the camera housing (16), the locking element (1821a, 1821b) slide in the openings, as the diameter of the other end of the locking element (1821a, 1821b) is same as the two legs (182a, 182b) and the openings, and the other end of the locking element (1821a, 1821b) which has bigger diameter get compressed and slid into the opening of the camera housing (16), thereby locking the deflector (18) on the camera housing (16). The deflector (18) which is detachable from the camera housing (16). Specifically, the deflector (18) is detachable from the camera housing (16) if the deflector (18) has incurred damage or requires replacement.

In another embodiment of the invention, the deflector (18) is an extruded portion (not shown) that extends from the outer surface of the camera housing (16). The extruded portion is provided between the image sensor opening (1611) and the light source opening (1621) to avoid direct light exposure to the image sensor (161) from the light source (162). The scattering of the light from the light source (162) makes images distorted and blurry. The deflector (18) avoids direct exposure of the light source (162) to the image sensor (161) to avoid distortion and blurriness of the images.

In another embodiment of the invention, the deflector (18) is a thin sheet arranged between the image sensor opening (1611) and the light source opening (1621). The sheet can be made up of any material that absorbs light to avoid the direct exposure of the light source (162) on the image sensor (161). It is obvious for the person skilled in the art to configure any shape of the deflector (18) with and material with any orientation to avoids direct exposure of the light source (162) to the image sensor (161) to avoid distortion and blurriness of the images.

Referring again to FIG. 1, and 2a, the handle (10) is adapted to receive a blade (20) from the lower end of the handle (10). The blade (20) is received around the outer surface of the handle (10) to entirely cover the handle (10) and the camera channel (15). The blade (20) is attached to the handle (10) while performing the intubation process. The handle is adapted to receive all types of blades (20) like Macintosh, and Miller. Further, the handle (10) is adapted to receive both adult blades and child blades. The camera channel (15) is adapted to receive all types of blades like Macintosh, and Miller for both adults and children. The handle (10) does not require any other adjustments or mountings to receive adult blades and child blades. The blade (20) is slidable over the camera channel (15) to attach with the handle (10) to perform the intubation process. The blade (20) is detachably attached to the handle (10) and is detachable from the handle (10) after the intubation process.

In the present embodiment of the invention, the blade (20) is attachable to the handle (10) through a dual ball mechanism (21) of the handle (10). The dual ball mechanism (21) includes two ball projections (211a, 211b) arranged opposite to each other on the outer surface of the handle adjacent to the dual projection (271a, 271b) to securely attach and detach the blade (20) to the handle (10). The dual ball mechanism (211a, 211b) is biased to radially extend away from the centre axis of the handle (10). The dual ball mechanism (211a, 211b) engages with the blade (20). Specifically, the blade (20) has two openings (not shown) to engage with two ball projections (211a, 211b) of the handle (10) to securely hold the blade (20) thereover. The two ball projections (211a, 211b) ensure that the blade (20) is held in place until the two ball projections (211a, 211b) are pressed to detach the blade (20).

The dual ball mechanism (21) having spring (not shown) attaché between each other, the dual ball mechanism (21) is biased and extended opposite to the central axis of the handle (10). Upon placing the blade (20) on the handle (10), the dual-ball mechanism (21) compresses towards the central axis of the handle (10), as the opening of the blade (20) aligned with the dual-ball mechanism (21), the dual-ball mechanism (21) extend away from the central axis of the handle (10) and engage with the opening to lock the blade (20) on the handle (10). To detach the blade (20) from the handle (10) and camera housing (16) of the laryngoscope (100), the clinician just needs to press the dual-ball mechanism (21) towards the central axis of the handle (10), and the dual-ball mechanism (21) will disengage from the opening of the blade (20) to detach the blade (20) from the laryngoscope (100).

The blade (20) is disposed of after detaching from the handle (10) to reduce the chance of infection and cross-contamination. Disposing of the blade (20) after one use allows the video laryngoscope (100) to control the spread of infection which can be spread by using the same blade (20) again and again.

As the top of the blade (20) is covering the handle (10) of the laryngoscope (100) and the bottom is covering the camera channel (15) and the camera housing (16). The shape of blade from the bottom end has same shape as the camera channel (15). The blade (20) has two-bottom ends, a first bottom end working as the guide to the laryngoscope (100) when the laryngoscope (100) is in the path of the endotracheal tube, larynx, trachea or airway, a second bottom end has an opening (165) having similar shape as the camera housing (16). The opening (165) allows the image sensor (161) and the light source (162) to capture the visuals of the path of the endotracheal tube. The image sensor (161) captures the visuals of the path of the endotracheal tube, larynx, trachea or airway and transfers the visuals to the display unit (30).

Before performing the intubation process, the clinicians attach the blade (20) to the handle (10). The blade (20) can be an adult blade or a child blade. The blade (20) is slidable over the camera channel (15) and is attached to the handle (10) through the dual ball mechanism (21) of the handle (10).

While performing the intubation process, the power button (32) is pressed to activate the display screen (34), the image sensor (161), and the light source (162). The visuals of the path of the endotracheal tube and the airway are displayed on the display screen (34). The deflector (18) of the camera housing (16) reduces the scattering of the light from the light source (162). The deflector (18) helps to reduce the distortion and blurriness of the images. Further, the record button (29) is provided to capture the image and record the video. The record button (29) is pressed and released to capture the image, and the record button (29) is pressed and held for 2 to 3 seconds to record the video. The video laryngoscope (100) is completely covered from the outside eliminating the risk of the video laryngoscope (100) getting infected. After the intubation process, the display cover (40) is removed from the display unit (30) and disposed of to avoid spreading infection. Further, the blade (20) is removed from the handle (10). The blade (20) which is mainly in contact with the body parts is disposed of after one use which helps to reduce the rate of infection or cross-contamination spread due to the repetitive use of the blade (20).

Further, the video laryngoscope (100) allows the clinician to have one hand free while operating the video laryngoscope (100) during the intubation process. In an operating room, clinicians need free hands to simultaneously use multiple devices or perform multiple actions. With the placement of the power button (32) at the bottom left of the display unit (30), the menu icon on the bottom left of the display screen (34) and the record button (29) under the display unit (30), the clinician can fully operate the video laryngoscope (100) with their left hand thus leaving the right hand free.

In another embodiment of the present invention, the video laryngoscope (100) includes a handle (10), a blade (20), and a display unit (30). The display unit (30) is attached to the handle (10) without using an attachment assembly (25). Specifically, the display unit (30) includes an attaching element (not shown) extending from a bottom portion of the display unit (30) to attach with the handle (10). It is obvious to a person skilled in the art to configure the attaching element of the display unit (30) to provide a threaded attachment or a snap-fit attachment or a similar attachment, to ensure a secure and functional connection between the display unit (30) and the handle (10).

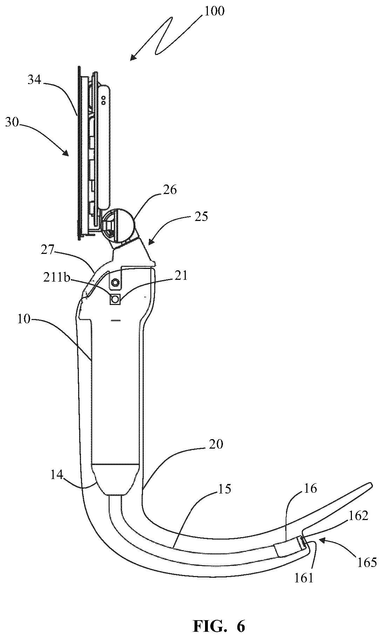

Referring now to FIG. 5 and 6, a second embodiment of the present invention. The display unit (30) is provided which is attachable to the handle (10) through the attachment assembly (25). The attachment assembly (25) includes a housing (27), and a second terminal (28) which is electrically connected to the display unit (30). The housing (27) is connected to the display unit (30) using a hinge (26). The hinge (26) is provided to pivot the display unit (30) in a back-and-forth direction. The hinge (26) is connected to the display unit (30) and the housing (27). Specifically, the hinge (26) includes a horizontal receptacle (not shown), and a horizontal shaft (not shown) is attached to the lower end of the display unit (30), the horizontal shaft and the horizonal receptacle are integrated which each other in a way that the horizontal shaft can rotate on a horizontal axis within the horizontal receptacle allowing the display unit (30) to adjust/move vertically. Further, the horizontal receptacle of the hinge (26) include a vertical shaft configured on the bottom centre portion, a top end of the vertical shaft is attached within a bottom centre portion of the horizontal receptacle in way that allows the vertical shaft to rotate within the bottom centre portion on a vertical axis, a lower end of the vertical shaft in integrated on the top end of the housing (27), the vertical shaft is in connection with the second terminal (28), to electrically connect to the display unit (30) with the second terminal (122).

The rotation of the vertical shaft on the vertical axis allows the display unit (30) to adjust horizontally on the vertical axis, thereby the hinge (26) allowing the display unit (30) to adjust vertically and horizontally as per the requirement. The housing (27) receives the vertical shaft of the hinge (26) to allow the display unit (30) to freely rotate around the central axis of the handle (10). The hinge (26) allows the display unit (30) to rotate in all the possible directions to configure the display unit (30) in a required position to provide better visibility to the clinician. The clinician can adjust the display unit (30) on the housing (27) in the vertical or the horizontal direction as per the requirement. In the present embodiment, the hinge (26) mechanism is arranged for allowing the display unit (30) to adjust at any required angle. It is obvious for the person skilled in the art to configure any other mechanism to allowing the adjustment of the display unit (30) at any orientation as per the requirement.

In another embodiment, a ball-and-socket joint may be arranged to allow rotation and movement in multiple directions as per the requirement. In another embodiment of the present invention, a universal joint may be arranged, the universal joint consists of two hinges connected by a cross shaft, allowing rotation and movement of the display unit (30) on two axes, providing adjustable display unit as per the requirement. In another embodiment of the present invention a gimbal mechanism may be arranged which is typically used for stabilizing cameras, allowing freedom of movement in three axes pitch, yaw, and roll. The gimbal mechanism will provide adjustable movement to the display unit (30) in all the angles.

In another embodiment of the present invention a swivel joint may be arranged between the display unit (30) and the housing (27) which enables display unit (30) to pivot horizontally and vertically around a central point. It is obvious for the person skilled in the art to configure any alternative mechanism to provide adjustable movement to the display unit (30) with respect to all the angles.

The handle (10) is configured to allow a clinician to hold the laryngoscope (100), the handle (10) has an upper end and a lower end. The upper end of the handle (10) is provided with a receiving port (12) with two openings (121a, 121b) opposite each other to receive the attachment assembly (25). The handle (10) is an elongated member that has an ergonomic design that allows clinicians to easily hold the handle (10) during an intubation process.

Further, the housing (27) includes dual projections (271a, 271b) having two ball projections (271a, 271b) extending from an outer surface of the housing (27). The dual projections (271a, 271b) are arranged opposite to each other having a compressible spring element (not shown) between each other which is arranged within the housing (27). The dual projections (271a, 271b) securely attach and detach the housing (27) to the handle (10). The dual projections (271a, 271b) are biased to radially extend away from the centre axis of the housing (27).

The housing (27) is attached to the receiving port (12) of the handle (10). Specifically, the receiving port (12) has two openings (121a, 121b) to receive the dual projections (271a, 271b) of the housing (27) to securely hold the housing (27) within the receiving port (12) of the handle (10). The dual projections (271a, 271b) ensure that the housing (27) is held in place until the dual projections (271a, 271b) are pressed to detach the housing (27) of the attachment assembly (25). The diameter of the handle (10) is bigger than the diameter of the housing (27), to securely receive the housing (27) with the top end of the handle (10).

Specifically, the dual projections (271a, 271b) have respective balls arranged at the extreme ends, and the dual projections (271a, 271b) include protruded rings arranged adjacent to the position of balls, upon placing the attachment assembly (25) within the handle (10), the dual projections (271a, 271b) compressed by the internal wall of the top end of the handle (10). The receiving ports (12) are arranged at predetermined distance from the top end of the handle (10), when the dual projections (271a, 271b) get aligned with the receiving ports (12), the dual projections (271a, 271b) extend radially opposite to the central axis of the housing (27) and pass through the receiving port (12) and the protruded ring engages with receiving port (120) of the handle (10), thereby securely attaching the display unit (30) to the handle (10). It is obvious for the person skilled in the art to configure any alternative mechanisms to attach the display unit (30) with the handle (10).

To detach the display unit (30) from the handle (10), the dual projections (271a, 271b) need to press towards the central axis of the housing (27), upon pressing the dual projection (271a, 271b) towards the central axis of the housing (27) the protruded rings disengages with the receiving ports (12) and the compressible spring element compressed towards the central axis of the housing (27) to disengages the dual projections (271a, 271b) from the receiving ports (12) of the handle (10) allowing the display unit (30) to detach from the handle (10).

In another aspect of the present invention, a bayonet mount mechanism may be arranged between the housing (27) and the top end of the handle (10) that features a pin or tab that locks into a slot via a twist-lock motion. The clinician can rotate one part to engage or disengage the handle (10) with the display unit (30).

In aspect of the present invention, a magnetic fastener may be arranged between the housing (27) and the top end of the handle (10) for attaching and detaching the display unit (30) from the handle (10). In another embodiment of the present invention, an interlocking tab may be arranged between a bottom end of the housing (27) and the upper end of the handle (10). The housing (27) has a flexible or rigid tabs and the top end of the handle (10) having a slot. The tabs snap fit into the slots for attaching the housing (27) to the handle (10) and to detach the housing (27) from the handle (10), the clinician has to press the tabs inwardly, thereby detaching the tabs from the slots of the handle (10) to release the housing (27) of the display unit (30).

In one more aspect of the present invention, a thread mechanism may be arranged between the housing (27) and the handle (10), allowing the clinician to attach and detach the housing (27) on the handle (10) by engaging and disengaging the thread mechanism. It is obvious for the persons skilled in the art to configure any mechanism not limited to disclosed above for attaching and detaching the housing (27) to the upper end of the handle (10) or attached with any other device having the receiving port (12).

Once the display unit (30) is attached to the handle (10) through the attachment assembly (25), the second terminal (28) of the attachment assembly (25) electrically connects with the handle (10) to establish the electric connection between the display unit (30) and the handle (10). Specifically, upon attaching the attachment assembly (25) to the handle (10), the second terminal (28) immediately establishes an electric connection between the display unit (30) and an image sensor (161) enabling the instantaneous display of visuals of the path of the endotracheal tube, larynx, trachea, or airway on the display screen (34).

Referring now to FIG. 7, and 8, a third embodiment of the present invention. The display unit (30) is provided which is attachable to top of the handle (10) through the attachment assembly (25). The handle (10) is configured to allow the clinician to hold the laryngoscope (100), the handle (10) includes a receiving port (12) with two openings (121a, 121b) opposite to each other. The handle (10) is an elongated member that has an ergonomic design that allows clinicians to easily hold the handle (10) during the intubation process.

The image sensor (161) is arranged on the camera housing (16) which is attached to the lower end of the handle (10) through the camera channel (15). The image sensor (161) facilitates capturing of the visuals of the path of the endotracheal tube, larynx, trachea or airway. Specifically, an image sensor opening (1611) provided in the camera housing (16) allows the image sensor (161) to capture the visuals of the path of the endotracheal tube, larynx, trachea or airway. In this embodiment, the image sensor (161) is a 2-megapixel (MP) camera, but the person skilled in the art can use any other image sensor (161) having different specifications to capture the visuals of the path of the endotracheal tube, larynx, trachea or airway.

Further, the camera housing (16) includes a light source opening (1621) to allow the light source (162) to illuminate the path of the endotracheal tube and the airway. Furthermore, the camera housing (16) has a deflector (18). Specifically, the deflector (18) is arranged on an outer surface of the camera housing (16) separating the image sensor opening (1611) and the light source opening (1621). The deflector (18) avoids the direct exposure of the light source (162) to the image sensor (161).

The display unit (30) is configured to show visuals of the path of the endotracheal tube, larynx, trachea, or airway. The display unit (30) is in a rectangular design having a protective edge made of flexible material to protect the display unit (30) from damage. It is obvious for the person skilled in the art to configure the display unit (30) of any shape or material as per the requirement. The display unit (30) includes a display screen (34) in the centre of the display unit (30). The display screen (34) is an LCD screen made of liquid crystal material. The display screen visualises images and videos through the conductors and electrode pattern, specifically the screen displays the visuals of the path of the endotracheal tube, larynx, trachea, or airway.

The display unit (30) further includes an output port (33). The output port (33) is provided on any of the sides of the display unit (30) to connect the display unit (30) to an external display (not shown) to show the visuals of the path of the endotracheal tube. The external display has a bigger screen size than the display screen (34) of the display unit (30). It is obvious to a person skilled in the art to provide a plurality of output ports on the display unit (30) to allow the clinician to connect the display unit (30) with plurality of the external display or use the output port as an USB or charging port to charge the battery of the display unit (32).

The display unit (30) further includes a processor (not shown), and a memory (not shown) to process and store the visuals of the path of the endotracheal tube, larynx, trachea or airway. The processor processes all the functions of the laryngoscope (100) including all the commands given by the clinician. The memory stores the files for future reference of the clinician. The person skilled in the art can configure any processor as per the requirement and also arrange memory with any capacity according to the requirement.

The display screen (34) further includes a touch user interface (TUI) (not shown) integrated within the display screen (34). The display screen (34) allows the clinician to interact with the laryngoscope (100). The touch user interface of the display screen (34) is adapted to review and manage previously recorded images or videos. The touch user interface includes a home page (not shown) to show the current time and date, a menu icon (not shown) to access the plurality of icons, and a battery icon (not shown) to indicate the level of the battery. The display screen (34) is powered on using the power button (32) to display the home page. The clinician interacts with the menu icon of the home page by touching the menu icon displayed on the display screen (34) to access the plurality of the icons. The menu icon is provided on the bottom left corner of the display screen (34) to allow the clinician to reach the menu icon with one hand.

The plurality of icons is assigned with specific actions to perform after receiving the command from the processor of the display unit (30). Specifically, the plurality of icons includes a settings icon (not shown) to make changes in the brightness of the display screen (34), the volume of the sound, turn on or turn off the output port (33), and the like. Further, the plurality of icons includes a photo icon (not shown) to view and manage previously recorded images, and a video icon to view and manage previously recorded videos. In another embodiment of the invention, the plurality of icons includes a folder icon (not shown) to view and manage the previously recorded images and videos in one place. A person skilled in the art can add as many icons as required associated with different functions as per the requirement.

In the present embodiment of the present invention, the display unit (30) includes a charging light (35) arranged on the top of the protective edge of the display unit to indicate the charging status of the battery. The charging light (35) turns ON when the battery is connected to a power source while recharge the battery, and turns OFF when the battery is not connected to the power source for recharging. It is obvious to a person skilled in the art to arrange the charging light (35) or any other element for indicating the charging status of the battery and can arranged that element to any of the corners of the display unit (30).

In the present embodiment, the display unit (30) automatically starts displaying visuals from the image sensor (161) when powered on and connected to the handle (10), eliminating the need to press the record button (29) to initiate the display of live visuals. The electric circuit between the display unit (30) and the camera housing (16) through the first terminal (122) at the bottom end of the handle (10) and the second terminal (28) at the top end of the handle (10), allows the display unit (30) to show and record the live visual without using the record bottom (29). It is obvious for the person skilled in the art to configure any alternative way to display the visual and record the visuals of the path of the endotracheal tube, larynx, trachea or airway.

Further, the clinician can capture still images with a single press and record a video with a press-and-hold action on the record button (29), with a visual timer displayed on the displaying unit during video recording. It is obvious for the person skilled in the art to configure the recode button (29) with multiple features to operate the display unit (30).

The display unit (30) further includes a security passcode (not shown), providing security to the previously stored file in the memory of the laryngoscope (100). The security passcode required for opening the file icon of the touch user interface. The clinician needs to put a key passcode in the security passcode in order to access the stored files within the memory. Further, the security passcode is also required upon connecting the display unit (30) with an external screen through the output port. The laryngoscope (100) initially comes with the default passcode which is "000000", the clinician can set any passcode within the 6 digits upon opening the setting icon of the security passcode.

In another embodiment of the present invention, the security passcode may be required for turning ON the laryngoscope (100). In another embodiment of the present invention, the security passcode may be required to transfer the recorded files from the memory of the laryngoscope (100) to another device. In another embodiment, a biometric authentication system (fingerprint, facial, iris or retinal, voice, palm or vein) may be arranged to access or transfer the recorded file. In another embodiment, a pattern mechanism may be arranged to access or transfer the recorded files. In another embodiment, two-factor authentication may be arranged within the display unit to access or transfer the recorded file. In another embodiment, a hardware security keys (USB or NFC keys), to access or transfer the recorded files. In another embodiment, behavioural biometrics (clinician's unique typing rhythm and speed, the way clinician move their mouse or interact with touchscreens) or a token-based authentication which uses a hardware or software token that generates unique codes for examples include RSA tokens or authenticator apps or gesture recognition may arranged to authenticates users through specific gestures made in front of the camera or using hand motions on a touch screen, or a proximity-based authentication may be arranged that uses Bluetooth to unlock a device when a trusted device (such as a smartphone or smartwatch) is near, or the like. It is obvious for the person skilled in the art to configure security passcode at any function of the laryngoscope (100) as per the requirement. Further, the person skilled in the art can also configure any security mechanism to secure the recorded files.

Moreover, the display screen (34) also includes an arrow (not shown) for adjusting the visual orientation while using the laryngoscope (100), the arrow feature allows for flipping visual orientation upside down as per the requirement of the situation. The clinician can interact with the display screen (34), and the arrow directing the adjustable direction is arranged on a top and bottom of the display screen (34). Upon interaction with the arrow, the commands go to the processor and the processor processes the commands and flips the display screen (34) visual vertically upside down. It is obvious for the person skilled in the art to configure any other way to adjust the visuals of the display screen (34) with any other icon not limited to the arrow, at any angle without disturbing the orientation of the display unit (30).

In the present embodiment of the invention, the display screen (34) is configured with the arrow that allows the adjustment of the display screen (34) to flip horizontally as per the requirement, without disturbing the orientation of the display unit (30).

In another embodiment of the present invention, the display screen (34) may be configured with the visual adjustment features without any icon such as arrow, the clinician can just interact with the display screen (34) with fingers and rotate the visual orientation with fingers. In another embodiment of the present invention, the laryngoscope (100) may be smart enough to take voice commands from the clinician to adjust the orientation of the display screen (34) visuals. It is obvious for the person skilled in the art to configure any features or icon or not limited to icon to adjust the visual orientation of the display screen (34) without disturbing the display unit (30) orientation.

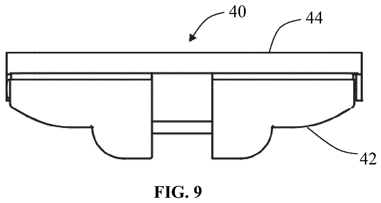

As shown in FIG. 9, a display cover (40) is provided to prevent the display unit (30) and the attachment assembly (25) from getting infected during the intubation process. The display cover (40) is made up of a transparent material to allow the clinician to view the display screen (34) without any hindrance.

Therefore, the present invention has the advantage of providing the video laryngoscope (100) to perform the intubation process. The video laryngoscope (100) is provided with the display unit (30) which rotates and moves in the back-and-forth direction to increase the visibility for the clinician. Further, the handle (10) of the video laryngoscope (100) is adapted to receive the adult blades as well as child blades, which reduces the other requirements like different handles for different blades or mountings to receive the different types of blades on the same handle (10). Further, the deflector (18) reduces the distortion and blurriness of the images for better quality visualisation. Furthermore, the handle (10) is covered by the blade (20) and the display unit (30) is covered by the display cover (40) which completely covers the video laryngoscope (100) and prevents the video laryngoscope (100) from getting infected during the intubation process.

The foregoing descriptions of specific embodiments of the present invention have been presented for purposes of illustration and description. They are not intended to be exhaustive or to limit the present invention to the precise forms disclosed, and obviously, many modifications and variations are possible in light of the above teaching. The embodiments were chosen and described in order to explain the principles of the present invention best and its practical application, to thereby enable others skilled in the art to best utilise the present invention and various embodiments with various modifications as are suited to the particular use contemplated. It is understood that various omission and substitutions of equivalents are contemplated as circumstance may suggest or render expedient, but such are intended to cover the application or implementation without departing from the scope of the claims of the present invention.

## Claims

1. A video laryngoscope (100) comprising:
a handle (10) having an upper end and a lower end configured to allow a clinician to hold the laryngoscope (100);
a display unit (30) attachable on the handle (10), the display unit (30) is attached on an upper end of the handle (10) through an attachment assembly (25);
a blade (20) removably attached around an outer surface of the handle (10), covering the entire handle (10) and the camera channel (15); and
a camera channel (15) extending from the lower end of the handle (10), the camera channel (15) is a flexible link having a first end connected to the handle (10) and a second end comprising a camera housing (16), the camera housing (16) adapted to capturing the visuals of the path of the endotracheal tube, larynx, trachea, or airway.

2. The video laryngoscope (100) as claimed in claim 1, wherein the attachment assembly (25) and the display unit (30) are connected through a hinge (26) mechanism, allowing 360-degree rotation of the display unit (30) for optimal viewing angles during the intubation procedure.

3. The video laryngoscope (100) as claimed in claim 1, wherein the blade (20) is attached to the handle (10) through a dual ball mechanism (21), the dual ball mechanism (21) includes two ball projections (211a, 211b) arranged opposite to each other and biased to radially extend away from the central axis of the handle (10), where the blade (20) includes two openings configured to engage with the respective ball projections (211a, 211b) of the handle (10), thereby securely attaching the blade (20) to the handle (10), and the two ball projections (211a, 211b) are configured to hold the blade (20) in place until the two ball projections (211a, 211b) are pressed towards the centre axis to detach the blade (20) from the handle (10).

4. The video laryngoscope (100) as claimed in claim 1, wherein the display unit (30) includes a touch user interface, adapted to allow the clinician to interact with stored images and videos, and to configure settings such as brightness, sound, and output port control.

5. The video laryngoscope as claimed in claim 1, wherein the display unit (30) is configured to establish a wireless connection with the handle (10), allowing the display unit (30) to display visuals from the image sensor (161) even when detached from the handle (10).

6. The video laryngoscope (100) as claimed in claim 1, wherein the handle (10) is adapted to receive both adult and paediatric blades, with no additional adjustments required, allowing for versatile use across different patient types during the intubation process.

7. The video laryngoscope (100) as claimed in claim 1, wherein the display unit (30) includes a display screen (34) for showing visuals of the path of the endotracheal tube, larynx, trachea, or airway, and a power button (32) to power ON or OFF the display unit (30).

8. The video laryngoscope (100) as claimed in claim 1, wherein the video laryngoscope (100) includes a memory and processor (not shown) to store and process captured images and videos, and an output port (33) for connecting the display unit (30) to an external display (not shown).

9. The video laryngoscope (100) as claimed in claim 1, wherein the display screen (34) is equipped with the arrow (not shown), allowing the clinician to rotate/flip the visuals of the display screen (34).

10. The video laryngoscope (100) as claimed in claim 1, wherein the display unit (30) is equipped with a passcode (not shown), the passcode is required to access the video or image stored in a memory, also the passcode is required to connect the display unit (30) to an external display such as a PC, Mac or Tablet or alike.

11. The video laryngoscope (100) as claimed in claim 1, wherein a first terminal (122) arranged within the lower end of the handle (10), the first terminal (122) is electrically connected with the camera housing (16), and a second terminal (28) is arranged at the lower end of the attachment assembly (25) electrically connected to the display unit (30), upon attaching the attachment assembly (25) on the handle (10), the second terminal (28) electrically connects with the first terminal (122) establishing the electric connection between the camera channel (15) and the display unit (30).

12. A video laryngoscope (100) comprising:
an attachment assembly (25);
a display unit (30) attached on an upper end of the attachment assembly (25) through a housing (27), adapted to display visuals of the path of the endotracheal tube, larynx, trachea, or airway, the housing (27) is arranged on the attachment assembly (25), the housing (27) includes dual projections (271a, 271b) extending from an outer surface, the dual projections (271a, 271b) being arranged opposite to each other and biased to radially extend away from a central axis of the attachment assembly (25);
a hinge (26) mechanism arranged between the housing (27) and the display unit (30), allowing 360-degree rotation of the display unit (30);
a handle (10) configured to allow a clinician to hold the laryngoscope (100), the handle (10) includes a receiving port (12) with two openings (121a, 121b) opposite to each other to receive the respective dual projections (271a, 271b) of the housing (27), thereby securely attaching the attachment assembly (25) to the handle (10), wherein, the dual projections (271a, 271b) are configured to hold the attachment assembly (25) in place within the receiving port (12) until the dual projections (271a, 271b) are pressed towards the central axis of the attachment assembly (25) to detach the attachment assembly (25) from the handle (10).

13. The video laryngoscope (100) as claimed in claim 12, wherein the dual projections (271a, 271b) include balls arranged on the extreme ends of the dual projection (271a, 271b), the dual projection (271a, 271b) are having protruded sleeves arranged adjacent to the position of the corresponding balls, while attaching the attachment assembly (25) to the handle (10), the protruded sleeve engages with the receiving port (12) of the handle (10), thereby securely attaching the display unit (30) to the handle (10).

14. A video laryngoscope (100) comprising:
a handle (10) configured to allow a clinician to hold the video laryngoscope (100); an image sensor (161) connected to the handle (10) and configured to capture the visuals of the path of the endotracheal tube, larynx, trachea or airway;
a display unit (30) attachable to the handle (10), the display unit (30) includes:
a display screen (34) for showing visuals of the path of the endotracheal tube, larynx, trachea, or airway, the display screen (34) includes a touch user interface, allowing the clinician to interact with the display screen (34), and an output port (33) for connecting the display unit (30) to an external display;
wherein, the display unit (30) is adapted to automatically start displaying visuals from the image sensor (161) when powered on and connected to the handle (10), eliminating the need to press the record button (29) to initiate the display of live visuals, and the display screen (34) is integrated with an arrow that allows the clinician to flip/rotate the visuals displayed on the display screen (34).

15. The video laryngoscope (100) as claimed in claim 14, wherein the display unit (30) includes a record button (29), the record button (29) is positioned such that the clinician can capture an image or record a video by pressing and releasing or holding the record button (29), respectively.
